# EUROPÄISCHE PATENTSCHRIFT

(11) **EP 1 649 874 B1**
(45) Veröffentlichungstag und Bekanntmachung des Hinweises auf die Patenterteilung: **21.09.2011**
(21) Anmeldenummer: 05020738.0
(22) Anmeldetag: 23.09.2005
(51) Int. Cl.: A61L 24/00, A61L 27/54, A61L 24/06, A61L 27/16

(54) **Antibiotikum-/Antibiotika enthaltender PMMA-Knochenzement**
PMMA bone cement containing antibiotics
Ciment osseux à base de PMMA contenant des antibiotiques

(30) Priorität: 07.10.2004 DE 102004049121
(43) Veröffentlichungstag der Anmeldung: 26.04.2006
(73) Patentinhaber: Heraeus Kulzer GmbH, 63450 Hanau (DE)
(72) Erfinder: Kühn, Klaus-Dieter, Dr., 35041 Marburg (DE); Vogt, Sebastian, Dr., 99084 Erfurt (DE)
(74) Vertreter: Kühn, Hans-Christian

(56) Entgegenhaltungen:
- EP-A- 0 581 387
- DE-A1- 19 918 295
- GB-A- 1 532 318
- US-A- 4 588 583
- US-A- 4 797 282
- US-A- 5 650 108
- US-A- 6 160 033
- US-A1- 2004 138 759
- US-B1- 6 689 823

## Beschreibung

Gegenstand der Erfindung ist ein Antibiotikum-/Antibiotika enthaltender PMMA-Knochenzement mit einer Pulverkomponente und einer flüssigen Komponente.

Antibiotika enthaltende PMMA-Knochenzemente (Polymethylmethavrylat-Knochenzemente) sind seit den sechziger Jahren des 20. Jahrhunderts basierend auf den Arbeiten von H. W. Buchholz und der Firma Kulzer bekannt (W. Ege, K.-D. Kühn: Industrial development of bone cement - 25 years of experience. In: bone Cement and Cementing Technique. Eds. G. H. I. M. Walenkamp, D. W. Murray, Springer Verlag Heidelberg 2001, in press; H. W. Buchholz, E. Engelbrecht: Über die Depotwirkung einiger Antibiotika beim Vermischen mit dem Kunstharz Palacos. Chirurg 41 (1970) 511-515). Diese PMMA-Zemente haben eine breite Akzeptanz gefunden und werden in großem Umfang zur Fixierung von Endoprothesen eingesetzt (K.-D. Kühn: Knochenzemente für die Endoprothetik: ein aktueller Vergleich der physikalischen und chemischen Eigenschaften handelsüblicher PMMA-Zemente. Springer-Verlag Berlin Heidelberg New York, 2001). Das in den PMMA-Knochenzement integrierte Antibiotikum wird nach der Implantation mehr oder weniger schnell an der Grenzfläche Knochenzement/Knochen lokal freigesetzt und soll dort die bakterielle Besiedlung verhindern. Angestrebt wird eine möglichst hohe initiale Freisetzung, so dass die minimal bakterizide Konzentration (MBK) des eingesetzten Antibiotikums gegenüber den klinisch relevantesten Keimen an der Grenzfläche Knochenzement/Knochen sicher erreicht und überschritten wird. Das in PMMA-Knochenzementen bisher am häufigsten eingesetzte Antibiotikum ist das breit wirksame Gentamicin.

US 6 160 033, das als relevantester Stand der Technik angesehen wird, offenbart PMMA-Zemente mit Antibiotikum-Partikeln und Propandiol. In diesem Dokument wird vorgeschlagen, dass 0.1-0.5 Masseprozent Antibiotikum-Partikel mit einem Partikeldurchmesser von 50-1000 Micrometern enthalten sind. Es gibt keinerlei Hinweise auf die Struktur der verwendeten Partikel.

Der Erfindung liegt die Aufgabe zu Grunde, einen PMMA-Knochenzement zu entwickeln, der eine sehr gute initiale Antibiotikum-/Antibiotika-Freisetzung zeigt. Das Antibiotikum soll innerhalb der ersten 24 Stunden nach der Aushärtung des Knochenzementes in hohen Mengen vom Knochenzement freigesetzt werden.

Die Aufgabe wird durch den Gegenstand des Anspruchs 1 gelöst. Vorteilhafte Ausgestaltungen sind in den Unteransprüchen angegeben. Der PMMA-Knochenzement ist dadurch charakterisiert, dass in der Pulverkomponente 0,1 - 5,0 Masseprozent in Wasser lösliche, glasartige Antibiotikum-/Antibiotika-Granula mit einem Partikeldurchmesser im Bereich von 50-1000 µm, bevorzugt 63 - 900 µm enthalten sind, die aus miteinander verbundenen, glasartigen Antibiotikum-/Antibiotika-Primär-partikeln aufgebaut sind, welche einen Partikeldurchmesser im Bereich von 1 - 70 µm haben.

Unter der Pulverkomponente des PMMA-Knochenzementes wird ein Gemisch aus mindestens einem pulverförmigen Polymethylmethacrylat oder einem Kopolymer, das aus Methylmethacrylat und Methylacrylat aufgebaut ist, einem pulverförmigen Röntgenopaker, wie Zirkoniumdioxid und/oder Bariumsulfat, und einem radikalischen Initiator, wie Dibenzoylperoxid, verstanden. Gegebenenfalls sind Bestandteile der Pulverkomponente mit einem pharmazeutisch akzeptablen Farbstoff eingefärbt. Die Pulverkomponente ergibt nach Vermischung mit der flüssigen Komponente, die aus Methylmethacrylat (MMA), in dem ein radikalischer Aktivator, wie N,N-Dimethyl-p-toluidin, gelöst ist, aufgebaut ist, einen plastisch verformbaren Teig, der nach wenigen Minuten selbständig durch die einsetzende radikalische Polymerisation des Methylmethacrylates aushärtet.

Unter dem Begriff glasartige Antibiotikum-/Antibiotika-Granula werden Granula aus einem oder mehreren Antibiotika verstanden, die keine lichtmikroskopisch erkennbare kristalline Struktur aufweisen und die transparent und/oder opak erscheinen. Die Antibiotikum-/Antibiotika-Granula haben ein glasartiges Erscheinungsbild. Weiterhin haben die Antibiotikum-/Antibiotika-Granula einen Partikeldurchmesser von ca. 50 -1000 µm und sind aus glasartigen, fest miteinander verbundenen Antibiotikum-/Antibiotika-Primärpartikeln aufgebaut. Unter dem Begriff fest miteinander verbundener, glasartiger Antibiotikum-/Antibiotika-Primärpartikel wird verstanden, dass die aus miteinander verbundenen Primärpartikel aufgebauten Granula so stabil sind, dass diese problemlos mit abrasiv wirkenden Röntgenopakem der Pulverkomponente, wie Zirkoniumdioxid und Bariumsulfat, vermahlen oder mit geeigneten Vorrichtungen vermischt werden können, ohne das ein signifikanter Zerfall der Granula in die Primärpartikel erfolgt. Glasartig bedeutet in diesem Zusammenhang ebenfalls, dass lichtmikroskopisch in den Primärpartikeln keine Kristalle erkennbar sind und dass die Primärpartikel selbst keine Kristalle darstellen. Weiterhin bedeutet der Begriff glasartig, dass die Primärpartikel transparent und/oder opak erscheinen.

Der erfindungsgemäß hergestellte PMMA-Knochenzement zeigte unter in vitro-Bedingungen bei 37 °C eine sehr hohe Antibiotikum-Freisetzung.

Vorteilhaft ist es, dass die Partikelgrenzen der glasartigen Primärpartikel nur an der Oberfläche der Antibiotikum-/Antibiotika-Granula lichtmikroskopisch erkennbar sind. Das bedeutet, lichtmikroskopisch kann aus der Oberflächenbeschaffenheit der Granula annähernd auf die Größe der Antibiotikum-/Antibiotika-Primärpartikel geschlossen werden.

Zweckmäßig ist es, dass die Antibiotikum-/Antibiotika-Granula aus mindestens einem Vertreter aus mindestens einer der Gruppen der Aminoglykosid-Antibiotika, der Lincosamid-Antibiotika, der Fluorchinolon-Antibiotika, der Glykopeptid-Antibiotika und der Nitroimidazole bestehen. Die antimikrobiell wirksamen Chemotherapeutika aus der Gruppe der Nitroimidazole werden vereinfacht auch als Antibiotika verstanden. Diese Chemotherapeutika wirken hauptsächlich bakterizid gegen anaerobe Keime.

Es ist zweckmäßig, dass die Antibiotikum-/Antibiotika-Granula bevorzugt aus Gentamicinsulfat, Gentamicinhydrochlorid, Amikacinsulfat, Amikacinhydrochlorid, Tobramycinsulfat, Tobramycinhydrochlorid, Clindamycinhydrochlorid, Lincosaminhydrochlorid, Moxifloxacin, Ciprofloxacin, Teicoplanin, Vancomycin, Ramoplanin, Metronidazol, Tinidazol oder Omidazol oder deren Gemischen bestehen. Neben diesen in Wasser löslichen Antibiotikasalzen und Antibiotika können auch in Wasser gering lösliche Salzformen der Antibiotika, wie beispielsweise Palmitate, Myristate und Laurate, zusätzlich in den Antibiotikum-/Antibiotika-Partikel integriert sein. Weiterhin ist es auch möglich, Antibiotika aus der Gruppe der Oxazolidone, wie Linezolid, in die Granula mit zu integrieren.

Vorteilhaft ist weiterhin, dass die Antibiotikum-/Antibiotika-Granula gegebenenfalls als Hilfsstoffe zusätzlich Polyvinylpyrrolidon und/oder Polyethylenglykol und/oder Polyethylenoxid und/oder Maltose und/oder Sorbitol und/oder Mannitol enthalten. Durch diese Hilfsstoffe können die Antibiotikum-/Antibiotika-Granula stabilisiert werden. Ebenfalls im Rahmen der Erfindung ist, das die Antibiotikum-/Antibiotika-Granula durch andere toxikologisch akzeptable Polymere, wie Gelatine, Kollagen und Dextran, stabilisiert sind. Im weiteren Sinne der Erfindung können von den erfindungsgemäßen Antibiotikum-/Antibiotika-Granula solche Granula abgeleitet werden, die aus Antibiotikum-/Antibiotika-Kristallen gebildet sind, die mit adhäsiven Hilfsstoffen zu Antibiotikum-/Antibiotika-Granula mit Partikelgrößen im Bereich von 50-1000 µm, bevorzugt 63-900 µm verklebt oder verkittet wurden.

Die Erfindung wird an zwei Beispielen erläutert ohne jedoch die Erfindung einzuschränken.

### Beispiel 1:

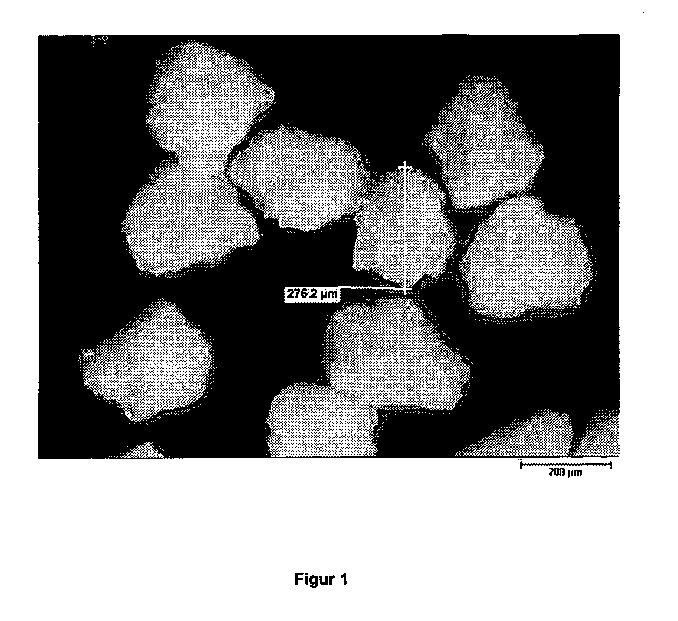

In der Figur 1 sind typische erfindungsgemäße Antibiotikum-Granula aus Gentamicinsulfat der Siebfraktion 125 - 250 µm abgebildet, wobei die Primärpartikel an der Oberflächenstruktur deutlich erkennbar sind.

### Beispiel 2:

Zur Prüfung des erfindungsgemäßen PMMA-Knochenzementes wurden Freisetzungsuntersuchungen an Probekörpern durchgeführt. Die Präparation der Probekörper erfolgte in der Weise, dass zuerst jeweils 40,0 g der Pulverkomponente des Knochenzementes Palacos ® (Heraeus Kulzer) mit

| | |
|---|---|
| Variante a) | 0,8 g Gentamicinsulfat der Siebfraktion < 63 µm |
| Variante b) | 0,8 g des aus Primärpartikeln aufgebaute, glasartige Gentamicinsulfat-Granulat der Siebfraktion 63 - 250 µm |

vermischt wurde. Danach wurden diese modifizierten Pulverkomponenten mit jeweils 20,0 g der Monomerkomponente vermischt. Es bildete sich ein grüner Teig, der in Hohlformen gestrichen wurde und dort nach wenigen Minuten aushärtete. Die entstandenen zylinderförmigen Probekörper hatten eine Höhe von 1 cm und einen Durchmesser von 2,5 cm. Es wurden jeweils 5 Probekörper pro Zementvariante hergestellt. Die Probekörper wurden separat in jeweils 20 ml destilliertem Wasser bei 37 °C eingelagert. Täglich wurde das Feisetzungsmedium vollständig entnommen und darin die freigesetzte Gentamicinmenge bestimmt. Die Probekörper wurden dann wieder in jeweils 20 ml frischem destillierten Wasser bei 37°C gelagert. Die Bestimmung des freigesetzten Gentamicins erfolgte mit einem TDX-Analyser der Firma Abott. Die jeweils freigesetzte Masse an Gentamicinbase wurde pro Gramm Probekörper in der nachfolgenden Tabelle in Abhängigkeit von der Lagerungszeit der Probekörper im Freisetzungsmedium angegeben.

| | freigesetzte Masse Gentamicinbase [µg/g] | | | |
|---|---|---|---|---|
| Lagerungszeit [d] | 1 | 2 | 3 | 4 |
| Variante a) | 113 | 6 | 4 | 0 |
| Variante b) | 217 | 33 | 17 | 11 |

## Patentansprüche

1. Antibiotikum-/Antibiotika enthaltender PMMA-Knochenzement mit einer Pulverkomponente und einer flüssigen Komponente, **dadurch gekennzeichnet, dass** in der Pulverkomponente 0,1 - 5,0 Masseprozent in Wasser lösliche, glasartige Antibiotikum-/Antibiotika-Granula enthalten sind, die aus mit einander verbundenen, glasartigen Antibiotikum-/Antibiotika-Primärpartikeln aufgebaut sind, welche einen Partikeldurchmesser im Bereich von 1 - 70 µm aufweisen.

2. PMMA-Knochenzement nach Anspruch 1, **dadurch gekennzeichnet, dass** die Antibiotikum-/Antibiotika-Granula Partikeldurchmesser im Bereich von 50 - 1000 µm aufweisen.

3. PMMA-Knochenzement nach Anspruch 1, **dadurch gekennzeichnet, dass** die Partikelgrenzen der glasartigen Antibiotikum-/Antibiotika-Primärpartikel nur an der Oberfläche der Antibiotikum-/Antibiotika-Granula lichtmikroskopisch erkennbar sind.

4. PMMA-Knochenzement nach Anspruch 1, 2 oder 3, **dadurch gekennzeichnet, dass** die Antibiotikum-/Antibiotika-Granula aus mindestens einem Vertreter aus einer der Gruppen der Aminoglykosid-Antibiotika, der Lincosamid-Antibiotika, der Fluorchinolon-Antibiotika, der Glykopeptid-Antibiotika oder der Nitroimidazole bestehen.

5. PMMA-Knochenzement nach einem der Ansprüche 1 bis 4, **dadurch gekennzeichnet, dass** die Antibiotikum-/Antibiotika-Granula aus Gentamicinsulfat, Gentamicinhydrochlorid, Amikacinsulfat, Amikacinhydrochlorid, Tobramycinsulfat, Tobramycinhydrochlorid, Clindamycinhydrochlorid, Lincosaminhydrochlorid, Moxifloxacin, Ciprofloxacin, Teicoplanin, Vancomycin, Ramoplanin, Metronidazol, Tinidazol oder Omidazol oder deren Gemischen besteht.

6. PMMA-Knochenzement nach einem der Ansprüche 1 bis 5, **dadurch gekennzeichnet, dass** die Antibiotikum-/Antibiotika-Granula als Hilfsstoffe zusätzlich Polyvinylpyrolidon und/oder Polyethylenglykol und/oder Polyethylenoxid und/oder Maltose und/oder Sorbitol und/oder Mannitol enthalten.

## Claims

1. PMMA bone cement containing antibiotic/antibiotics, with a powder component and a liquid component, **characterised in that** the powder component contains 0.1 - 5.0% by weight water-soluble, glass-like antibiotic/antibiotics granules made of glass-like antibiotic/antibiotics primary particles bonded to each other which have a particle diameter in the range of 1-70 µm.

2. PMMA bone cement according to claim 1 **characterised in that** the antibiotic/antibiotics granules have particle diameters in the range of 50 - 1000 µm.

3. PMMA bone cement according to claim 1 **characterised in that** the particle boundaries of the glass-like antibiotic/antibiotics primary particles are recognisable by light microscopy only at the surface of the antibiotic/antibiotics granules.

4. PMMA bone cement according to claim 1, 2 or 3 **characterised in that** the antibiotic/antibiotics granules consist of at least one representative from one of the groups of aminoglycoside antibiotics, lincosamide antibiotics, fluoroquinolone antibiotics, glycopeptide antibiotics or nitroimidazoles.

5. PMMA bone cement according to any one of claims 1 to 4 **characterised in that** the antibiotic/antibiotics granules consist of gentamicin sulphate, gentamicin hydrochloride, amikacin sulphate, amikacin hydrochloride, tobramycin sulphate, tobramycin hydrochloride, clindamycin hydrochloride, lincosamine hydrochloride, moxifloxacin, ciprofloxacin, teicoplanin, vancomycin, ramoplanin, metronidazole, tinidazole or omidazole or mixtures thereof.

6. PMMA bone cement according to any one of claims 1 to 5 **characterised in that** the antibiotic/antibiotics granules additionally contain polyvinyl pyrrolidone and/or polyethylene glycol and/or polyethylene oxide and/or maltose and/or sorbitol and/or mannitol as excipients.

## Revendications

1. Ciment osseux de PMMA contenant un antibiotique/des antibiotiques avec un composant pulvérulent et un composant liquide, **caractérisé en ce que** 0,1 à 5,0 pourcent en masse de granules d'antibiotique/antibiotiques vitreux, solubles dans l'eau sont contenus dans le composant pulvérulent, lesquels sont construits à partir de particules primaires d'antibiotique/antibiotiques vitreux reliées entre elles qui présentent un diamètre particulaire dans la plage de 1 à 70 µm.

2. Ciment osseux de PMMA selon la revendication 1, **caractérisé en ce que** les granules d'antibiotique/antibiotiques présentent un diamètre particulaire dans la plage de 50 à 1000 µm.

3. Ciment osseux de PMMA selon la revendication 1, **caractérisé en ce que** les limites des particules primaires d'antibiotique/antibiotiques vitreux ne sont reconnaissables qu'à la surface des granules d'antibiotique/antibiotiques par microscopie optique.

4. Ciment osseux de PMMA selon la revendication 1, 2 ou 3, **caractérisé en ce que** les granules d'antibiotique/antibiotiques se composent d'au moins un représentant d'un des groupes des antibiotiques d'aminoglycoside, des antibiotiques de lincosamide, des antibiotiques de fluoroquinolone, des antibiotiques de glycopeptide ou des nitroimidazoles.

5. Ciment osseux de PMMA selon l'une quelconque des revendications 1 à 4, **caractérisé en ce que** les granules d'antibiotique/antibiotiques se composent de sulfate de gentamicine, de chlorhydrate de gentamicine, de sulfate d'amicacine, de chlorhydrate d'amicacine, de sulfate de tobramycine, de chlorhydrate de tobramycine, de chlorhydrate de clindamycine, de chlorhydrate de lincosamine, de moxifloxacine, de ciprofloxacine, de teicoplanine, de vancomycine, de ramoplanine, de métronidazol, de tinidazol ou d'omidazol ou de leurs mélanges.

6. Ciment osseux de PMMA selon l'une quelconque des revendications 1 à 5, **caractérisé en ce que** les granules d'antibiotique/antibiotiques contiennent en outre en tant que substances auxiliaires de la polyvinylpyrrolidone et/ou du polyéthylèneglycol et/ou de l'oxyde de polyéthylène et/ou du maltose et/ou du sorbitol et/ou du mannitol.
